# FASCICULE DE BREVET EUROPEEN

(11) **EP 1 651 293 B1**
(45) Date de publication et mention de la délivrance du brevet: **29.11.2006**
(21) Numéro de dépôt: 04743958.3
(22) Date de dépôt: 15.07.2004
(51) Int. Cl.: A61M 5/315

(54) **DISPOSITIF D EJECTION D UN PRODUIT LIQUIDE OU PATEUX**
VORRICHTUNG ZUM AUSDRÜCKEN EINER FLÜSSIGEN ODER PASTÖSEN SUBSTANZ
DEVICE FOR EXPELLING A LIQUID OR PASTY SUBSTANCE

(30) Priorité: 16.07.2003 FR 0308686
(43) Date de publication de la demande: 03.05.2006
(73) Titulaire: Weill, David, 1268 Begnins (CH)
(72) Inventeur: VOLCKMANN, Jean-Claude, F-74100 Cret (Ville-La-Grand) (FR); ROSSET, Yannick, CH-1298 Céligny (CH)
(74) Mandataire: Bugnion Genève
(86) Numéro de dépôt international: PCT/IB2004/002297
(87) Numéro de publication internationale: WO 2005/007224

(56) Documents cités:
- FR-A- 2 535 206
- US-A- 4 444 560
- US-A- 4 693 684
- US-A- 4 820 287
- US-A- 5 891 106

## Description

L'invention concerne un dispositif d'éjection d'un produit liquide ou pâteux, comprenant un corps, une partie destinée à contenir le produit et munie d'un orifice pour l'éjection du produit, un cylindre de poussée muni de dents, se déplaçant dans un alésage traversant le corps, et faisant varier le volume de la partie destinée à contenir le produit et un mécanisme de déplacement du cylindre de poussée lié au corps, comprenant un levier articulé agissant sur les dents du cylindre de poussée par le biais d'un cliquet articulé sur le levier et rappelé dans une position de contact avec le cylindre de poussée et un cliquet anti-retour rappelé dans une position de contact avec le cylindre de poussée.

Ce type de dispositif est connu du brevet US 4,444,560 ou de la demande de brevet FR 2 535 206 qui décrit une seringue dentaire pour injection intra-ligamentaire. Cette seringue permet l'injection par une aiguille très fine et souple de produit dans les ligaments situés entre l'os de la mâchoire et la dent. Elle est principalement constituée d'un corps allongé sur lequel est monté un mécanisme commandant l'injection par le biais du déplacement d'un cylindre de poussée, d'un porte-conteneur dans lequel est logé un conteneur rempli de liquide à injecter et d'un embout comportant l'aiguille d'injection. Afin de résoudre des problèmes d'accès difficile aux zones où doivent être faites les injections, le corps de la seringue présente une tête d'injection qui fait un angle avec l'axe du corps de la seringue. L'aiguille, amovible, est mise en place sur le corps avant de pratiquer les injections puis retirée après. Le mécanisme commandant l'injection est composé principalement d'un levier articulé sur le corps de seringue et agissant sur un cylindre de poussée par le biais d'un cliquet articulé sur le levier et rappeler dans une position de contact avec les dents d'une crémaillère réalisées sur le cylindre de poussée. Le cylindre est guidé en translation dans un alésage réalisé dans le corps de seringue. Il présente en outre une rainure longitudinale coopérant avec une vis vissée radialement par rapport à l'alésage et débouchant dans celui-ci pour interdire la rotation du cylindre. Le mécanisme présente de plus un cliquet anti-retour interdisant le recul du cylindre de poussée lorsqu'on met fin à l'action sur le levier. Ce cliquet anti-retour est rappelé dans une position de contact avec les dents de la crémaillère et peut être éloigné de cette position par action sur un bouton pour annuler la pression d'injection et/ou pour changer le conteneur de produit à injecter sur lequel appuie le cylindre de poussée.

Un tel dispositif présente des inconvénients. D'une part, sa réalisation est complexe. D'autre part, il présente de nombreuses pièces et des formes complexes, en particulier, des angles et des coins dans la matière. Ces angles et coins forment des zones très peu accessibles et, par conséquent, très difficiles à nettoyer et donc difficiles à stériliser.

Le but de l'invention est de proposer un dispositif d'éjection d'un produit liquide ou pâteux palliant aux inconvénients cités et améliorant les dispositifs connus de l'état de la technique. En particulier, l'invention propose de réaliser un dispositif présentant une construction simple, aisément démontable et nettoyable.

Le dispositif d'éjection selon l'invention est caractérisé en ce que le levier est lié au corps au moyen d'un emboîtement démontable. Cette caractéristique permet d'assurer un accès aisé pour le nettoyage des différentes pièces du dispositif.

Différents modes de réalisation du dispositif sont définis par les revendications dépendantes 2 à 10.

Le dessin annexé représente, à titre d'exemples, deux modes de réalisation d'un dispositif d'éjection de produit selon l'invention.

La figure 1 est une vue d'un mode de réalisation d'un dispositif d'éjection de liquide selon l'invention.

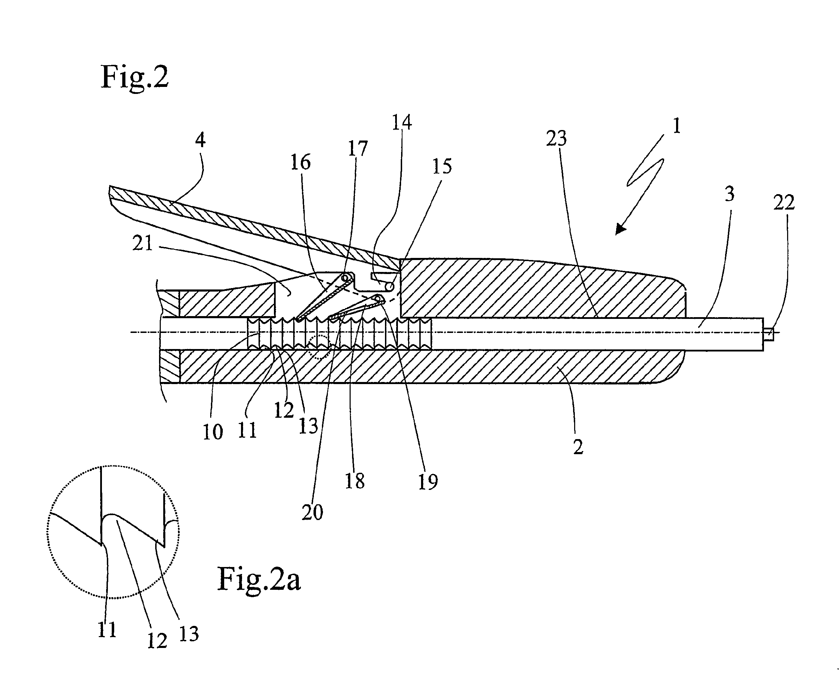

La figure 2 est une vue en coupe de ce mode de réalisation.

La figure 3 est une vue d'une première variante de réalisation du cylindre de poussée.

La figure 4 est une vue d'une seconde variante de réalisation du cylindre de poussée.

Les figures 5 et 6 sont des vues en coupe de la seconde variante de réalisation du cylindre de poussée.

La figure 7 est une vue en coupe d'un deuxième mode de réalisation d'un dispositif d'éjection de liquide selon l'invention.

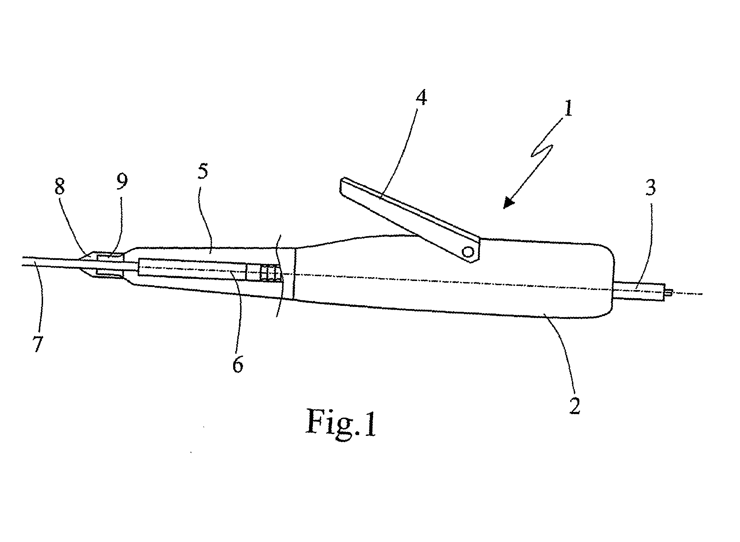

Le dispositif 1 d'éjection de produit représenté à la figure 1 comprend principalement un corps 2 sur lequel est fixé un porte-conteneur 5.

Le porte-conteneur 5 présente une cavité cylindrique 6 destinée à recevoir soit un conteneur rempli de produit à éjecter, soit directement le produit à éjecter. Le fond du porte-conteneur présente un canal dans lequel est fixé de manière amovible un tube 7 lié par exemple à une bague taraudée 8 ou à une bague présentant un autre système de fixation. Cette fixation est réalisée par vissage de la bague taraudée sur un embout fileté 9 réalisé sur le porte-conteneur autour du canal.

Le corps 2 comprend un mécanisme d'éjection et de dosage de produit. Ce mécanisme présente un levier 4 articulé sur le corps autour d'un axe 15. Ce levier est lié au corps par emboîtement par déformation élastique de son axe 15 dans des fentes 14 réalisées dans le corps 2. Cet emboîtement assure un démontage aisé du levier qui ne nécessite de préférence pas d'utiliser un outil de démontage et permet par conséquent l'accès pour le nettoyage du levier et des régions du corps situées sous le levier. Il permet en outre à l'utilisateur de monter sur le corps un levier dont la taille est adaptée à sa morphologie. Ceci permet d'améliorer la précision de manipulation du dispositif. Ce levier est par exemple constitué par un profilé en U dont les deux ailes latérales sont percées pour permettre le passage de l'axe 15. Le levier permet d'agir sur un cylindre de poussée 3 réalisé par exemple en acier inoxydable et présentant des dents 10 pouvant avoir une symétrie de révolution et dont le profil est constitué de deux segments rectilignes 11 et 13 raccordés par un rayon 12. Un tel profil de dent permet d'éviter de créer des zones d'accessibilité délicate et difficiles à nettoyer. En particulier, le rayon de fond de dent présente une dimension suffisante pour minimiser les risques de dépôt de salissures au fond des dents et pour faciliter l'accès du matériel de nettoyage. La valeur du rayon est par exemple de l'ordre de grandeur de la profondeur des dents. Les deux segments pourront présenter, avec l'axe du cylindre, des angles permettant eux aussi de faciliter l'accès du matériel de nettoyage. Le cylindre 3 est guidé en translation dans le corps 2 par un alésage 23. Son extrémité localisée dans la cavité cylindrique permet de repousser soit une paroi du conteneur rempli de produit à éjecter, soit directement le produit à éjecter. L'alésage 23 débouche de part et d'autre du corps 2. Il est ainsi parfaitement accessible et ne présente pas de région difficilement nettoyable.

Le mécanisme d'éjection comprend un cliquet 16 démontable, articulé sur le levier 4 autour d'un axe 17 et rappelé par un ressort non représenté dans une position de contact avec les dents du cylindre 3. Cet effort de rappel permet d'assurer un contact permanent entre le cliquet 16 et le cylindre, d'une part, et d'assurer le rappel du levier 4 dans une position éloignée du corps 2 depuis laquelle il est possible d'exercer une action de nature à éjecter une partie du produit contenu dans le porte-conteneur.

Le mécanisme d'éjection comprend en outre un cliquet anti-retour 18 articulé sur le corps 2 autour d'un axe 19 et rappelé par un ressort non représenté dans une position de contact avec les dents du cylindre 3. Ce cliquet anti-retour permet d'éviter le recul du cylindre de poussée, lorsque l'utilisateur relâche la pression qu'il exerce sur le levier 4 afin que celui-ci revienne dans sa position éloignée du corps. Ce cliquet anti-retour peut additionnellement présenter une ouverture 20 coopérant avec une saillie 22 prévue à l'une des extrémités du cylindre pour interdire l'introduction de celui-ci à l'intérieur de l'alésage dans le mauvais sens. Le mécanisme d'éjection pourrait encore présenter une structure telle que le levier agit directement sur les dents du cylindre de poussée.

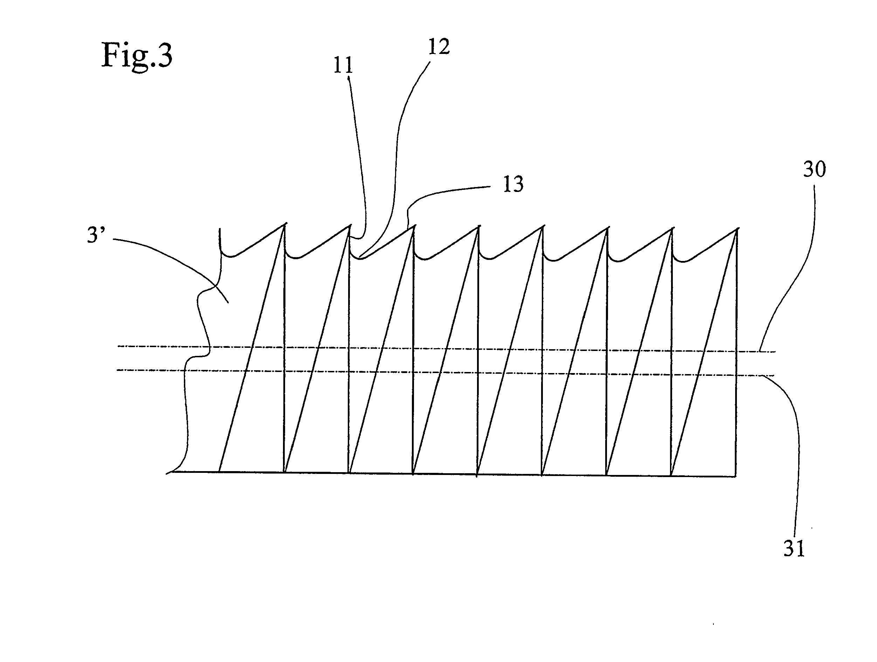

Dans une première variante de réalisation du cylindre de poussée 3', représentée à la figure 3, les dents ont une symétrie de révolution mais la distance de l'axe de révolution 31 du profil au profil est supérieur au rayon du cylindre. De cette façon, le cylindre de poussée présente un secteur dépourvu de dent sur toute sa longueur. Ceci permet d'annuler rapidement la pression dans le porte-conteneur par rotation du cylindre de poussée.

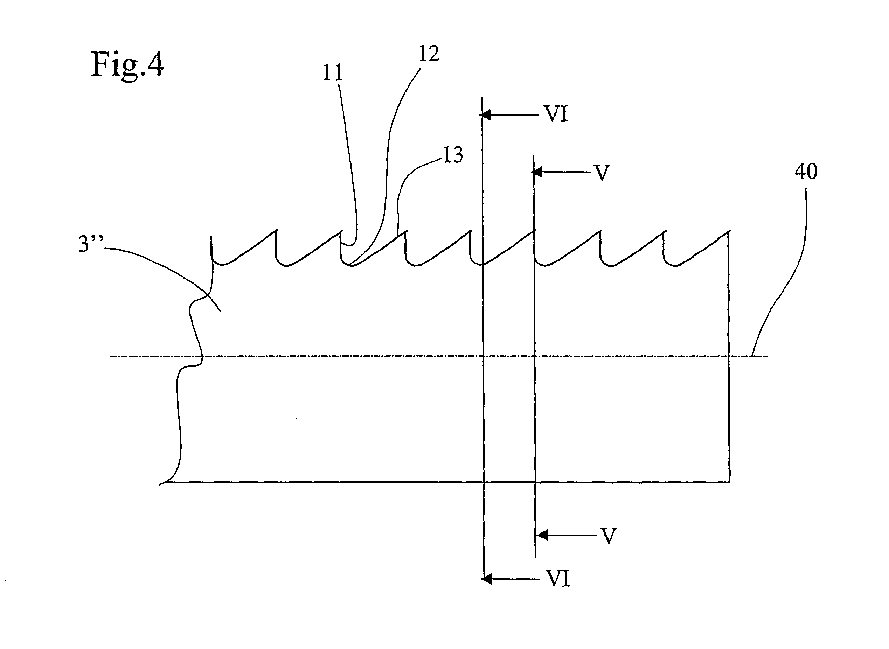

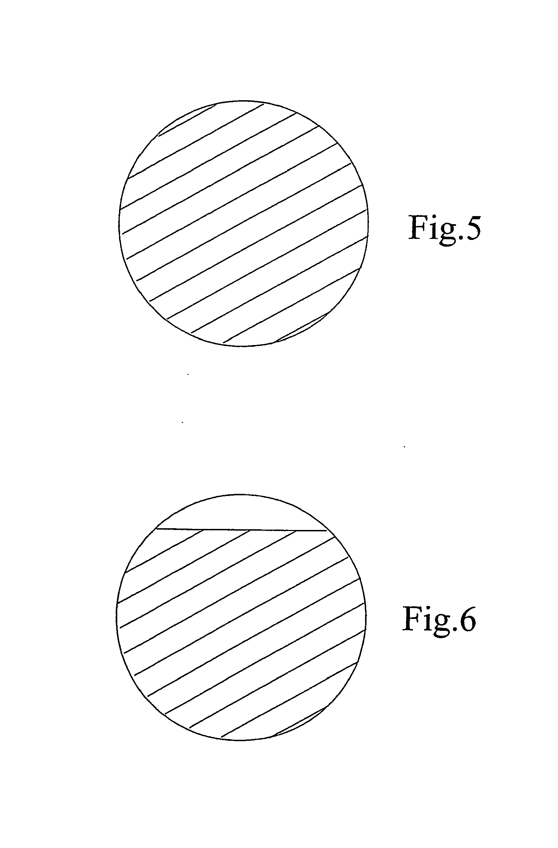

Dans une deuxième variante de réalisation du cylindre de poussée 3", représentée à la figure 4, les dents constituent une surface réglée dont les génératrices sont, au niveau des dents, sensiblement orthoradiales au cylindre de poussée. Le cylindre de poussée présente ainsi également un secteur dépourvu de dent sur toute sa longueur et permet l'annulation rapide de la pression dans le porte-conteneur par rotation du cylindre de poussée. La section du cylindre est représentée au niveau d'un sommet de dent à la figure 5 et au niveau d'un creux de dent à la figure 6.

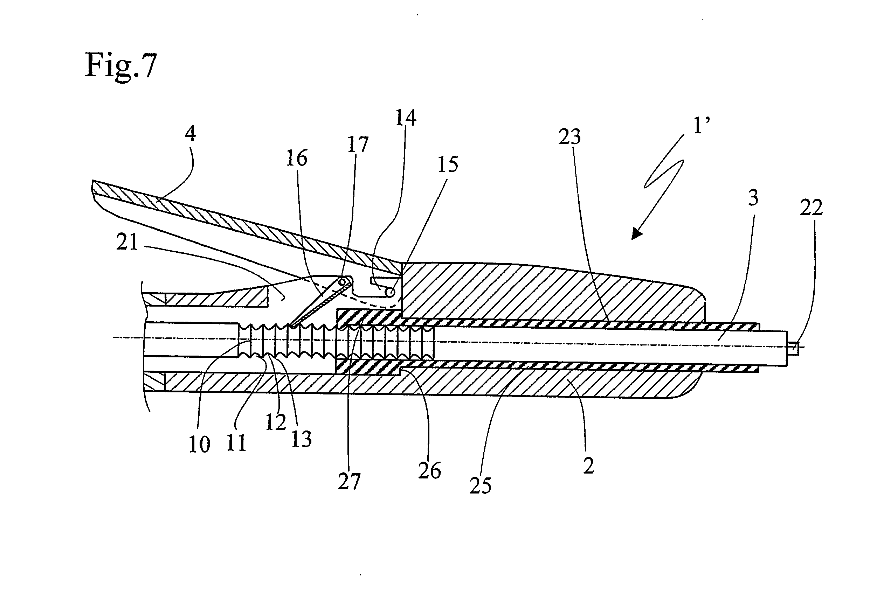

Un deuxième mode de réalisation du dispositif selon l'invention est représenté à la figure 7. Ce mode de réalisation diffère du précédent en ce que l'alésage 23 présente un épaulement 26 au niveau de la lumière 21 réalisée dans le corps 2. L'alésage reçoit une chemise 25 réalisée par exemple en polytétrafluoroéthylène ou un autre matériau plastique tel qu'un polyétheréthercétone (PEEK). Cette chemise comprend, sur son diamètre extérieur, un épaulement coopérant avec l'épaulement 26 de l'alésage pour l'arrêter en translation. Cette chemise est montée glissante dans le corps. Elle comprend au niveau de la lumière 21 deux fentes axiales débouchant à son extrémité pour réaliser un ergot 27 dont l'extrémité vient en contact avec les dents 10 du cylindre de poussée. L'ergot est rappelé dans cette position par les forces de déformation élastique du matériau le constituant. Un tel mode de réalisation permet de simplifier la construction du dispositif et, ainsi, de le rendre plus facilement nettoyable. Avec une telle structure, pour annuler la pression d'éjection du produit, il n'est plus nécessaire de tourner le cylindre de poussée par rapport au corps. En effet, il suffit de tourner le cylindre de poussée par rapport à la chemise. Ce mouvement pouvant notamment être obtenu par rotation de la chemise par rapport au corps, le cylindre de poussée étant fixe par rapport au corps. La rotation de la chemise par rapport au corps est plus aisée que celle du cylindre de poussée. En effet, les efforts appliqués à l'extrémité du cylindre de poussée peuvent être tels que ceux-ci combinés au coefficient de frottement entre le cylindre de poussée et le conteneur rendent impossible cette rotation. Le cliquet anti-retour peut être constitué d'une partie déformable du corps comme il peut être constitué d'une partie déformable de la chemise.

La chemise peut, dans des variantes de réalisation, présenter des ergots de libération du cylindre de poussée sur son diamètre extérieur et être mobile en translation ou en rotation par rapport au corps. Ces mouvements permettent aux ergots de libération d'agir sur les différents cliquets pour les amener dans une position libérant le cylindre de poussé. La chemise peut également présenter des moyens lui permettant d'assurer la liaison entre le corps et la partie destinée à contenir le produit. Par exemple, elle peut présenter à l'une de ses extrémités des ergots pour lui permettre d'être assemblée à la partie destinée à contenir le produit par une liaison à baïonnette et présenter à l'autre de ses extrémités un épaulement maintenant le corps contre la partie destinée à contenir le produit.

Les matériaux constituant les différentes pièces du dispositif peuvent être choisis parmi les produits compatibles avec les procédés de stérilisation.

Un tel dispositif peut être utilisé dans le domaine médical pour l'injection de produits tels que des anesthésiques dans des tissus durs ou pour le dépôt de colles, de résines ou d'amalgames. Il peut également être utilisé dans le domaine paramédical pour déposer des quantités déterminées de collagène. Il peut en outre être utilisé dans le domaine de la micromécanique et de la bijouterie pour effectuer des collages ou des microsoudures ou encore pour déposer des produits.

## Revendications

1. Dispositif (1) d'éjection d'un produit liquide ou pâteux, comprenant un corps (2), une partie destinée à contenir le produit et munie d'un orifice pour l'éjection du produit, un cylindre de poussée (3 ; 3' ; 3") muni de dents (10), se déplaçant dans un alésage (23) traversant le corps, et faisant varier le volume de la partie destinée à contenir le produit et un mécanisme de déplacement du cylindre de poussée lié au corps, comprenant un levier (4) articulé agissant sur les dents du cylindre de poussée par le biais d'un cliquet articulé (16) sur le levier (4) et rappelé dans une position de contact avec le cylindre de poussée et un cliquet anti-retour (18) rappelé dans une position de contact avec le cylindre de poussée, **caractérisé en ce que** le levier (4) est lié au corps au moyen d'un emboîtement démontable (14, 15).

2. Dispositif (1) d'éjection selon la revendication 1 **caractérisé en ce que** le cylindre de poussée (3 ; 3' ;3") présente une extrémité (22) conformée pour s'engager dans une lumière (20) pratiquée sur le cliquet anti-retour (18) lorsque celui-ci est engagé dans le corps (2) dans le mauvais sens.

3. Dispositif (1) d'éjection selon l'une des revendications précédentes, **caractérisé en ce que** le cylindre de poussée (3' ; 3") présente un secteur dépourvu de dent sur toute sa longueur et est mobile en rotation dans l'alésage (23).

4. Dispositif (1) d'éjection selon l'une des revendications précédentes, **caractérisé en ce que** le profil des dents (10) entre deux sommets consécutifs comprend deux segments droits (11, 13) raccordés par un rayon (12).

5. Dispositif (1) d'éjection selon l'une quelconque des revendications 1 à 4, **caractérisé en ce qu'**il comprend, dans le corps (2), une chemise (25) mobile en rotation par rapport au corps.

6. Dispositif (1) d'éjection selon la revendication 5, **caractérisé en ce que** la chemise comprend une partie élastiquement déformable (27) constituant le cliquet anti-retour.

7. Dispositif (1) d'éjection selon la revendication 5 ou 6, **caractérisé en ce que** la chemise comprend au moins un ergot agencé pour agir sur au moins un cliquet et libérer le cylindre de poussée lors d'un mouvement de rotation de la chemise.

8. Dispositif (1) d'éjection selon l'une quelconque des revendications 1 à 4, **caractérisé en ce qu'**il comprend, dans le corps (2), une chemise (25) mobile en translation par rapport au corps (2) et **en ce que** la chemise comprend au moins un ergot agencé pour agir sur au moins un cliquet et libérer le cylindre de poussée lors d'un mouvement de translation de la chemise.

9. Dispositif (1) d'éjection selon l'une quelconque des revendications 5 à 8, **caractérisé en ce que** la chemise comprend un moyen de liaison à la partie destinée à contenir le produit.

10. Dispositif (1) d'éjection selon l'une quelconque des revendications précédentes, **caractérisé en ce que** le corps (2) et/ou le levier (4) sont réalisés en un matériau moulable.

## Claims

1. A device (1) for ejecting a liquid or pasty product, comprising a body (2), a part intended to contain the product and equipped with an orifice for ejecting the product, a drive cylinder (3; 3'; 3") equipped with teeth (10), moving in a bore (23) passing through the body and causing the volume of the part intended to contain the product to vary and a mechanism for displacing the drive cylinder attached to the body, comprising an articulated lever (4) acting on the teeth of the drive cylinder through an articulated pawl (16) articulated to the lever (4) and returned to a position of contact with the drive cylinder and a nonreturn pawl (18) returned to a position of contact with the drive cylinder, **characterized in that** the lever (4) is connected to the body by means of a joint (14, 15) that can be dislocated.

2. The ejection device (1) as claimed in claim 1, **characterized in that** the drive cylinder (3; 3'; 3") has one end (22) shaped to engage in a slot (20) formed on the nonreturn pawl (18) when the latter is engaged the wrong way round in the body (2).

3. The ejection device (1) as claimed in one of previous claims, **characterized in that** the drive cylinder (3'; 3'') has a sector with no teeth along its entire length and is able to rotate in the bore (23).

4. The ejection device (1) as claimed in one of previous claims, **characterized in that** the profile of the teeth (10) between two consecutive crests comprises two straight segments (11, 13) connected by a radius (12).

5. The ejection device (1) as claimed in one of claims 1 to 4, **characterized in that** it comprises, in the body (2), a liner (25) able to rotate with respect to the body.

6. The ejection device (1) as claimed in claim 5, **characterized in that** the liner comprises an elastically deformable part (27) constituting the nonreturn pawl.

7. The ejection device (1) as claimed in claim 5 or 6, **characterized in that** the liner comprises at least one stud designed to act on at least one pawl and to release the drive cylinder as the liner turns.

8. The ejection device (1) as claimed in one of claims 1 to 4, **characterized in that** it comprises, in the body (2), a liner (25) capable of translational movement with respect to the body (2) and **in that** the liner comprises at least one stud designed to act on at least one pawl and release the drive cylinder as the liner effects a translational movement.

9. The ejection device (1) as claimed in one of claims 5 to 8, **characterized in that** the liner comprises a means of connection to the part intended to contain the product.

10. The ejection device (1) as claimed in one of previous claims, **characterized in that** the body (2) and/or the lever (4) are made of a material that can be molded.

## Patentansprüche

1. Vorrichtung (1) zur Abgabe einer flüssigen oder pastösen Substanz; mit einem Körper (2); mit einem Bereich zur Aufnahme der Substanz, der mit einer Öffnung zur Abgabe der Substanz versehen ist; mit einem Schubzylinder (3; 3'; 3") mit Zähnen (10), der in einer Bohrung (23) des Körpers verschiebbar ist und das Volumen des Bereiches, der zur Aufnahme der Substanz bestimmt ist, verändert; und mit einem mit dem Körper verbundenen Mechanismus zum Verschieben des Schubzylinders, der einen angelenkten Hebel (4) aufweist, welcher mit Hilfe einer am Hebel (4) gelenkig angeordneten Klinke (16) auf die Zähne des Schubzylinders einwirkt und eine Rückholstellung in Berührung mit dem Schubzylinder einnimmt, und mit einer Rücklauf-Sperrklinke (18), die eine Rückholstellung in Berührung mit dem Schubzylinder einnimmt, **dadurch gekennzeichnet, dass** der Hebel (4) mittels einer demontierbaren Einhängepassung (14, 15) mit dem Körper verbunden ist.

2. Abgabevorrichtung (1) nach Anspruch 1, **dadurch gekennzeichnet, dass** der Schubzylinder (3; 3'; 3") einen Endbereich (22) aufweist, der so ausgebildet ist, dass er in eine Aussparung (20) eingreifen kann, welche an der Rücklauf-Sperrklinke (18) angebracht ist, wenn letztere in der falschen Richtung im Körper (2) liegt.

3. Abgabevorrichtung (1) nach einem der vorstehenden Ansprüche, **dadurch gekennzeichnet, dass** der Schubzylinder (3', 3") einen Abschnitt aufweist, der über seine ganze Länge ohne Zähne ausgeführt ist und in der Bohrung (23) drehbeweglich ist.

4. Abgabevorrichtung (1) nach einem der vorstehenden Ansprüche, **dadurch gekennzeichnet, dass** das Profil der Zähne (10) zwischen zwei aufeinanderfolgenden Gewindespitzen zwei geradlinige Segmente (11, 13) aufweist, welche durch einen Radius (12) miteinander verbunden sind.

5. Abgabevorrichtung (1) nach einem der Ansprüche 1 bis 4, **dadurch gekennzeichnet, dass** in den Körper (2) ein Futter (25) eingesetzt ist, welches gegenüber dem Körper drehbeweglich ist.

6. Abgabevorrichtung (1) nach Anspruch 5, **dadurch gekennzeichnet, dass** das Futter einen elastisch deformierbaren Bereich (27) aufweist, der die Rücklauf-Sperrklinke bildet.

7. Abgabevorrichtung (1) nach Anspruch 5 oder 6, **dadurch gekennzeichnet, dass** das Futter mindestens einen Zapfen aufweist, welcher zur Einwirkung auf mindestens eine Klinke und zur Freigabe des Schubzylinders bei einer Drehung des Futters eingerichtet ist.

8. Abgabevorrichtung (1) nach einem der Ansprüche 1 bis 4, **dadurch gekennzeichnet, dass** in den Körper (2) ein gegenüber diesem Körper (2) längsbewegliches Futter (25) eingesetzt ist, und dass das Futter mindestens einen Zapfen aufweist, welcher zur Einwirkung auf mindestens eine Klinke und zur Freigabe des Schubzylinders bei einer Längsbewegung des Futters eingerichtet ist.

9. Abgabevorrichtung (1) nach einem der Ansprüche 5 bis 8, **dadurch gekennzeichnet, dass** das Futter ein Mittel zur Verbindung mit dem Bereich aufweist, der zur Aufnahme der Substanz bestimmt ist.

10. Abgabevorrichtung (1) nach einem der vorstehenden Ansprüche, **dadurch gekennzeichnet, dass** der Körper (2) und/oder der Hebel (4) aus einem verformbaren Material erzeugt sind.
